# EUROPÄISCHE PATENTSCHRIFT

(11) **EP 0 531 666 B1**
(45) Veröffentlichungstag und Bekanntmachung des Hinweises auf die Patenterteilung: **13.01.1999**
(21) Anmeldenummer: 92112119.0
(22) Anmeldetag: 15.07.1992
(51) Int. Cl.: B29C 65/08, A61F 13/15

(54) **Verfahren und Vorrichtung zum Verbinden von Einzelteilen bei höschenartigen Kleidungsstücken**
Process and apparatus for joining parts of trouserlike clothing
Procédé et appareil pour la liaison d'éléments de vêtement en forme de culotte

(30) Priorität: 15.07.1991 JP 199999/91
(43) Veröffentlichungstag der Anmeldung: 17.03.1993
(73) Patentinhaber: UNI-CHARM CORPORATION, Kawanoe-shi Ehime-ken (JP)
(72) Erfinder: Nomura, Hironori, Iyomishima-shi, Ehime-ken (JP); Takahashi, Tatsuo, Iyomishima-shi, Ehime-ken (JP); Yamamoto, Hiroki, Mitoyo-gun, Kagawa-ken (JP)
(74) Vertreter: Sperling, Rüdiger, Dipl.-Ing.

(56) Entgegenhaltungen:
- EP-A- 234 658
- EP-A- 417 766
- DE-A- 1 804 988
- FR-A- 1 008 847
- US-A- 3 939 033
- US-A- 4 117 180
- US-A- 4 333 782
- US-A- 4 373 979
- US-A- 4 427 485
- US-A- 4 650 530

## Beschreibung

Die Erfindung betrifft ein Verbindungsverfahren in einem kontinuierlichen Herstellungsprozeß für höschenartige Kleidungsstücke sowie eine Ultraschall-Schweißeinrichtung für einen kontinuierlichen Herstellungsprozeß von höschenartigen kleidungsstücken.

Bei der Herstellung eines höschenartigen Kleidungsstücks war es bisher in der Praxis üblich, daß die jeweiligen Lagenelemente, die Vorder- und Hinterabschnitte bilden, übereinander gelegt werden und anschließend entlang den seitlich gegenüberliegenden Hüftseiten miteinander verbunden werden. Beispielsweise kann ein einzelnes Lagenelement, das sowohl den Vorder- wie auch den Hinterabschnitt umfaßt, entlang einer Linie im Schrittbereich übereinander gefaltet werden und entlang den seitlich gegenüberliegenden Hüftseiten verbunden werden, um eine Grundkonfiguration des Höschens zu bilden. Dieses Verbinden wird oftmals durch einzelne oder mehrere durchgehende Verbindungslinien aus Heißschmelzkleber, Verbindungsnähten o.ä. erzielt.

Das so hergestellte höschenartige Kleidungsstück bietet eine gute Passform und verhindert in wirksamer Weise das Austreten von Exkrementen. Ein derartiges Produkt nach dem Stand der Technik hat jedoch eine relativ geringe Luftdurchlässigkeit und führt dadurch zur Ansammlung von Feuchtigkeit. Darüber hinaus weist dieses Produkt den Nachteil auf, daß nach der Aufnahme von Körperflüssigkeiten oder Exkrementen die Beine des Trägers leicht mit der Körperflüssigkeit oder den Exkrementen beschmutzt werden, wenn das Kleidungsstück entlang den Beinen abgetreift wird. Diese Nachteile können vermieden werden, indem die seitlich gegenüberliegenden Hüftseiten des höschenartigen Kleidungsstückes punktweise miteinander verbunden werden. Dabei können der Vorder- und Hinterabschnitt des Kleidungsstückes übereinander gelegt und anschließend entlang den gegenüberliegenden Hüftseiten mit Unterbrechungen verbunden werden, um so luftdurchlässige Lücken zwischen dem Vorder- und Hinterabschnitt zu lassen. Darüberhinaus können die einander gegenüberliegenden Hüftseiten ohne weiteres entlang den Verbindungslinien aufgerissen werden, wodurch das Kleidungsstück dem Träger ohne Beschmutzung der Beine abgenommen werden kann.

Während in einem kontinuierlichen Herstellungsprozess für die höschenartigen Kleidungsstücke eine punktweise Verbindung bzw. eine Verbindung mit Unterbrechungen entlang den seitlich gegenüberliegenden Hüftseiten mit geeignetem Klebstoff unter Verwendung bekannter Klebetechniken einfach ist, kann der ausgehärtete Kleber die Haut des Trägers reizen. Das Verbinden bzw. Befestigen mittels Nähten ist durch die geringe Arbeitsgeschwindigkeit und die relativ hohen Kosten nachteilhaft. Die Verwendung von Ultraschall-Schweißtechniken wurde zwar bereits angeregt, es ist jedoch noch kein bestimmtes Verfahren zur Verwendung in einem kontinuierlichen Herstellungsprozess des Kleidungsstückes aufgezeigt worden.

Aus der US-A.-4,650,530 ist ein gattungsgemäßes Verfahren zur Herstellung von höschenartigen Bekleidungsstücken bekannt, bei dem zur Herstellung von Verbindungslinien zwischen vorderen und hinteren Abschnitten des Kleidungsstückes ein Ultraschallverfahren eingesetzt wird. Die dabei zum Einsatz kommende Ultraschall-Schweißeinrichtung besitzt mehrere Schallköpfe und mehrere Stempel, die den Schallköpfen gegenüberliegend angeordnet sind. Zur Führung und Steuerung der Schallköpfe und der Stempel ist eine komplexe Mechanik vorgesehen. Da diese bekannte Vorrichtung zur Herstellung von Turnhosen dient, besitzen die bekannten Stempel und Schallköpfe lineare Kontaktflächen, die zur durchgehenden und damit luftundurchlässigen Verbindungslinien führen. Dieses bekannte Verfahren kann daher nicht zur Herstellung von Verbindungslinien mit einer gewissen Luftdurchlässigkeit verwendet werden, da auch ein Aufreißen der Verbindungslinien, die bei diesem Stand der Technik kontinuierlich durchgehend ausgebildet sind, nicht möglich ist.

Aus der US-A-4,117,180 ist die Herstellung eines Faltenbandes bekannt, das aus zwei Lagen eines ultraschallverschweißten Lagenmaterials besteht, das gewebt oder nicht gewebt sein kann, wobei die Querverschweißungslinien Faltenlinien bilden, an denen das Band faltbar ist. In Längsrichtung ist das Band zum Zwecke des Versteifens ebenfalls ultraschallverschweißt.

Aus der EP-A-0 234 658 ist ein adhäsivfreies Verfahren zum Verbinden eines sich kontinuierlich fortbewegenden Gewebes bekannt, während in diesem Verfahren eine Laminatlagenbahn sowie ein aus dieser Bahn herausgeschnittenes Produkt bekannt ist. Hierbei werden ebenfalls Ultraschallschweißverfahren verwendet, wobei ein stationäres Vibrationshorn und eine relativ weich bewegliche Arbeitslage zwischen der zu verarbeitenden Lage und dem Horn geführt wird. Hierdurch wird die Qualität und die Wirksamkeit der Verschweißung erhöht und gleichzeitig vermieden, daß an der herzustellenden Lage Schäden auftreten.

Aus der US-4,427,485 ist ein Verfahren zum Herstellen einer ultraschallgeschweißten schraubenförmig hergestellten Schlauchanordnung bekannt, wobei der biegsame Schlauch an einem schraubenförmigen Draht geformt und hauptsächlich aus einem Plastikband mit überlappender Wicklungen gebildet wird. Diese Überlappungen unmittelbar sind ultraschallverschweißt, ohne daß zusätzlich Klebstoff verwendet wird. Die Vorrichtung weist einen Dorn auf, auf den das Band mit überlappenden Rändern aufgewickelt ist, über welche die Ultraschallverschweißung ausgeführt wird.

Aus der US-4333782 ist ein Verfahren zum Herstellen einer laminierten Struktur mit gerafften und ungerafften Randabschnitten bekannt. Hierbei weist die Laminatstruktur ein plastisches Teil zwischen zwei ersten und zweiten Substraten eines flexiblen, faltbaren Materials auf, wobei das elastische Teil eine Vielzahl von in Längsrichtung sich erstreckenden Elementen besitzt, die über einen Teil ihrer Länge miteinander verbunden sind.

Schließlich ist aus der EP-A-0 417 766 ein Verfahren zum Herstellen eines Wegwerf-Kleidungsstückes bekannt, wobei Innenteile dadurch gebildet werden, daß ein Absorptionskörper an innere Lagen befestigt wird und in Längsrichtung ein vorbestimmter Abstand zwischen den Kernen verbleibt. Die inneren Teile werden entlang ihrer Mittellinie so gefaltet, daß die Kerne teilweise auf einer ersten äußeren Oberfläche und teilweise auf einer zweiten äußeren Oberfläche, welche der ersten Oberfläche zugewandt ist, zu liegen kommen. Einige solcher kontinuierlichen Lagen mit einer Breite, die die erste und die zweite Oberfläche bedecken kann, ist mit elastischen Teilen für Beinöffnungen und einer Hüftöffnung versehen und mit der ersten und zweiten Oberfläche verbunden, um eine kontinuierliche Laminatkonstruktion zu schaffen. Das Laminat ist sukzessive versiegelt und wird an vorbestimmten Stellen geschnitten und so die individuellen Wergwerf-Kleiderstücke geschaffen.

### BESCHREIBUNG DER ERFINDUNG

Demgemäß ist es Aufgabe der Erfindung, die bei vorstehend beschriebenem Stand der Technik auftretenden Nachteile zu vermeiden, indem eine Ultraschall-Schweißeinrichtung verwendet wird, die mit einer mit Stempeleinrichtungen versehenen Walze ausgerüstet ist, basierend auf der Erkenntnis, daß Ultraschall-Schweißstellen die Reizung der Haut des Trägers möglichst gering halten können und das Erzielen von gewünschten, punktweisen Verbindungen erleichtern können. Vorstehende Aufgabe wird erfindungsgemäß durch ein Verbindungsverfahren gemäß Anspruch 1 gelöst.

Weiter wird die Aufgabe durch eine Ultraschall-Schweißeinrichtung gemäß Anspruch 3 gelöst.

Zweckmäßige Weiterbildungen der Erfindung sind in den abhängigen Ansprüchen beschrieben.

Bei diesem Verbindungsverfahren enthalten sowohl die obere Lage als auch die untere Lage thermisch verschweißbares Material. Eine Ultraschall-Schweißeinrichtung zur Durchführung des Befestigungsvorganges weist einen Schall- bzw. Schweißkopf auf, dessen Länge im wesentlichen der Länge der jeweiligen Verbindungslinie, die zwischen den gegenüberliegenden Hüftseiten ausgebildet werden soll, sowie den Stempeleinrichtungen entspricht, wobei der Schallkopf in Querrichtung zur Zulieferrichtung der oberen und unteren Lage ausgerichtet ist und die Stempeleinrichtung dem Schallkopf gegenüberliegend an der Umfangsfläche einer Walze angeordnet ist, die in derselben Richtung drehbar ist, in der die obere und untere Lage zugeliefert werden. Die Stempeleinrichtungen umfassen mehrere Vorsprünge, die beabstandet angeordnet sind, so daß sie einem vorgegebenen Verbindungsmuster entsprechen. Die Vorder- und Hinterabschnitte, die übereinander gelegt sind, werden der Ultraschall-Schweißeinrichtung koninuierlich zugeliefert. In einer bevorzugten Ausführungsform enthält das Kleidungsstück elastische Elemente, die um eine Hüftöffnung und/oder Beinöffnungen angebracht sind.

Merkmale der Ultraschall-Schweißeinrichtung für einen kontinuierlichen Herstellungsprozeß von höschenartigen kleidungsstücken sind, daß ein Schallkopf der Ultraschall-Schweißeinrichtung im wesentlichen dieselbe Länge wie die jeweiligen Verbindungslinien aufweist, die entlang den jeweiligen gegenüberliegenden Hüftseiten auszubilden sind, und quer zur Zulieferrichtung der oberen und unteren Lage angeordnet ist, und daß dem Schallkopf gegenüberliegend Stempeleinrichtungen auf der Umfangsfläche einer in Förderrichtung der oberen und unteren Lage drehbaren Walze angeordnet sind, die eine Vielzahl von Vorsprüngen umfassen, die im Abstand zueinander angeordnet sind, so daß sie einem vorgegebenen Verbindungsmuster entsprechen. In einer bevorzugten Ausführungsform sind wenigstens zwei Stempeleinrichtungen symmetrisch und in einem bestimmten Winkel bezüglich der Walzenachse angeordnet. In einer weiteren bevorzugten Ausführungsform ist ein Abstand zwischen benachbarten Vorsprüngen in einem vorgegebenen Bereich der Stempeleinrichtungen größer als die Breite der elastischen Elemente, die in einen solchen Abstand eingelegt werden.

Sowohl die wasserdurchlässige obere Lage als auch die wasserundurchlässige untere Lage enthalten thermisch verschweißbares Material und die Vorder- und Hinterabschnitte, die diese obere und untere Lage in übereinandergelegtem Zustand umfassen, können der Ultraschall-Schweißeinrichtung kontinuierlich zugeführt werden, um in kontinuierlicher Weise die gewünschten Verbindungslinien zu erhalten, wobei jede der Verbindungslinien ein punktweises bzw. unterbrochenes Verbindungsmuster aufweist und so einen die Haut nicht reizenden Griff ergibt.

Soll das Kleidungsstück mit elastischen Elementen um die Hüftöffnung und/oder die Beinöffnungen versehen werden, so werden die elastischen Elemente jeweils zwischen zwei benachbarten Vorsprüngen auf der Walze angeordnet, wobei sichergestellt ist, daß nur die obere und untere Lage zwischen dem Schallkopf und den Stempeleinrichtungen gepreßt werden.

Auf der Umfangsfläche der drehbaren Walze sind zwei Stempeleinrichtungen in symmetrischer Anordnung bezüglich der Achse der Walze angeordnet, wodurch es bezüglich jedes Paares von benachbarten einzelnen Kleidungsstücken im kontinuierlichen Herstellungsprozeß erleichtert ist, zwei unmittelbar benachbarte Verbindungslinien entlang den aneinanderliegenden Hüftseiten benachbarter einzelner Kleidungsstücke zu bilden.

Der Schallkopf ist parallel zur Walzenachse angeordnet, während die Stempeleinrichtung in einem Winkel bezüglich der Walzenachse angeordnet ist, so daß ein Bereich, in dem der Schallkopf quer gegen die Stempeleinrichtung drückt, relativ verkleinert wird, wodurch die für einen ordnungsgemäßen Schweißvorgang erforderliche Last am Schallkopf entsprechend verringert ist.

### KURZE BESCHREIBUNG DER FIGUREN

Die Erfindung wird im Detail unter Bezug auf die beiliegenden Figuren erläutert. Es zeigen:
- Fig. 1: eine perspektivische Darstellung eines einzelnen höschenartigen Kleidungsstückes;
- Fig. 2: eine auseinandergezogene Darstellung dieses Kleidungsstückes;
- Fig. 3: eine schematische Draufsicht eines kontinuierlichen Prozesses zur Herstellung der Kleidungsstücke;
- Fig. 4: die Seitenansicht einer Ultraschall-Schweißeinrichtung;
- Fig. 5: eine Draufsicht auf die Stempeleinrichtungen;
- Fig. 6A, 6B u. 6C: Seitenansichten der Stempeleinrichtungen; und
- 7A, 7B: perspektivische Darstellungen von Beispielen für Verbindungslinien, die entlang den seitlich gegenüberliegenden Hüftseiten ausgebildet sind.

### BEVORZUGTE AUSFÜHRUNGSFORMEN DER ERFINDUNG

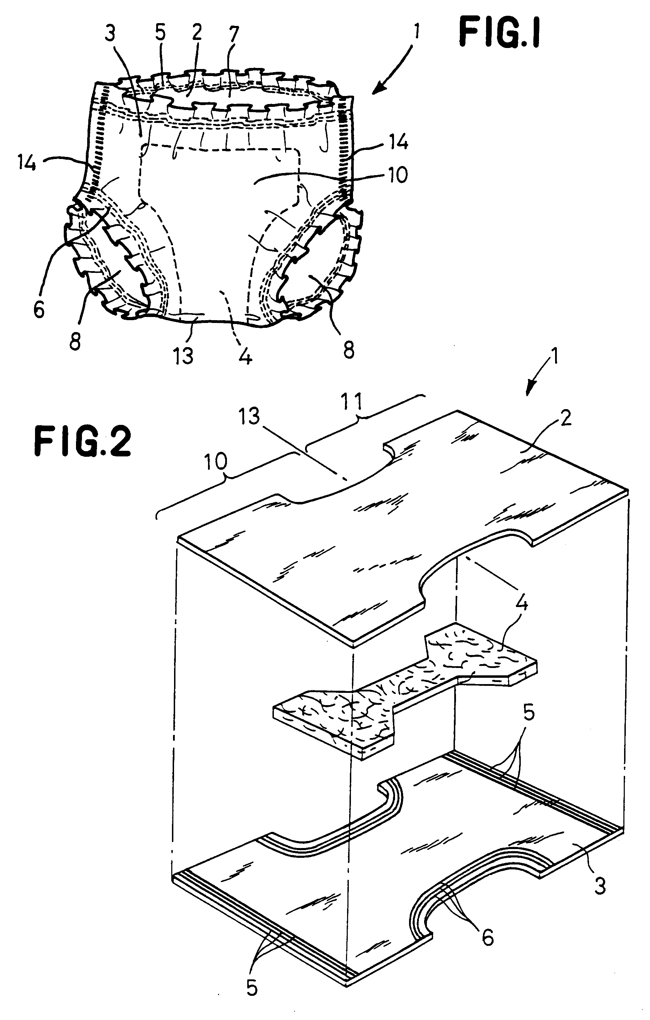

Fig. 1 und 2 zeigen perspektivische Darstellungen eines höschenartigen Kleidungsstückes 1, das nach dem erfindungsgemäßen Verfahren hergestellt ist, bzw. eine auseinandergezogene perspektivische Darstellung des Kleidungsstückes 1, das die Anordnung der Einzelteile in Richtung der Dicke darstellt.

Das Kleidungsstück 1 umfaßt eine flüssigkeitsdurchlässige obere Lage 2, eine flüssigkeitsundurchlässige untere Lage 3 und einen Absorbtionskern 4, der zwischen diese Lagen gelegt ist. Diese Lagen werden entlang einer Schrittlinie 13 mit nach innen weisender oberer Lage 2 übereinandergefaltet, so daß ein Vorderabschnitt 10 und ein Hinterabschnitt 11 gebildet werden. Das Kleidungsstück 1 verfügt über eine Hüftöffnung 7 und über zwei Beinöffnungen 8. Ein bandähnliches elastisches Element 5 ist punktweise in gestrecktem Zustand mit der Innenfläche der unteren Lage 3 um die Hüftöffnung 7 verklebt und entsprechende bandähnliche elastische Elemente 6 sind um die jeweiligen Beinöffnungen 8 angeordnet. Der Absorbtionskern 4 ist punktweise mit der unteren Lage 3 verklebt, die wiederum punktweise mit der oberen Lage 2 in Bereichen verbunden ist, in denen die beiden Lagen 2 und 3 miteinander in Berührung stehen.

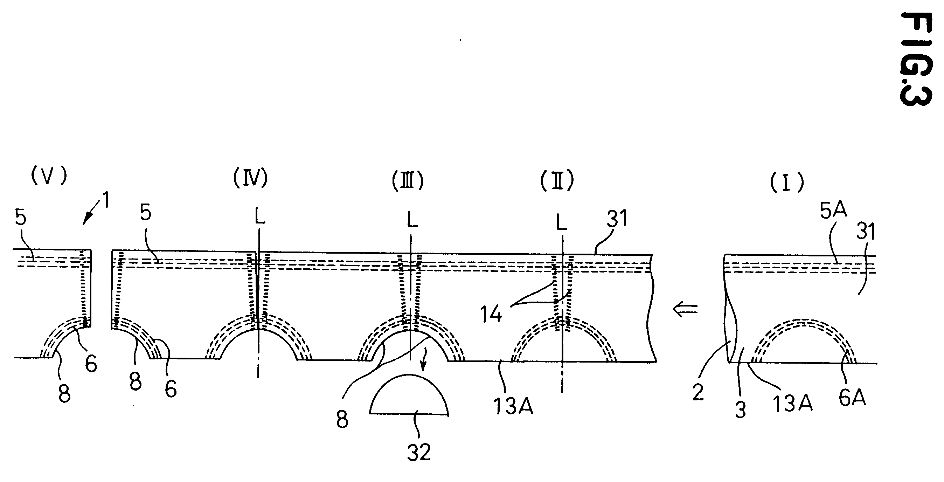

Fig. 3 ist eine schematische Darstellung eines Teiles des kontinuierlichen Herstellungsprozesses des Kleidungsstückes 1, bei dem eine übereinandergefaltete Endlosbahn 31, die den Vorderabschnitt 10 und den Hinterabschnitt 11 bilden soll, mit Verbindungslinien 14 und den Beinöffnungen 8 versehen wird. Beim hier dargestellten Prozeß wurde die Endlosbahn 31 entlang einer in Längsrichtung verlaufenden Linie 13a so übereinandergefaltet, daß die obere Lage 2 nach innen weist und die untere Lage 3 nach außen. Die Linie 13A entspricht der Schrittlinie 13 des Kleidungsstückes 1. Der zwischen der oberen und unteren Lage 2, 3 liegende Absorbtionskern 4 ist nicht dargestellt. Auf die Innenfläche der unteren Lage 3 ist ein elastisches Element 6A punktweise in gestrecktem Zustand in der Weise aufgeklebt, daß es eine halbkreisförmige Kurve beschreibt, die von einem entlang der Linie 13A verlaufenden Durchmesser bestimmt ist, und ein geradliniges elastisches Element 5A ist punktweise ebenfalls in gestrecktem Zustand entlang einem Seitenrand parallel zur Linie 13A aufgeklebt. Die Endlosbahn 31 ist in Schritt (I) unterbrochen dargestellt. In Schritt (II) wird die Endlosbahn 31 mit paarweise angeordneten Verbindungslinien 14 versehen, die bezüglich einer Linie L symmetrisch angeordnet sind, die den vom elastischen Element 6A beschriebenen Halbkreis teilt. In Schritt (III) wird ein Abschnitt 32, dessen Begrenzung geringfügig innerhalb des vom elastischen Element 6A beschriebenen Halbkreises liegt, aus der Endlosbahn 31 ausgeschnitten, um so einen den jeweils benachbarten Beinöffnungen 8 entsprechenden Ausschnitt zu bilden. In Schritt (IV) wird die Endlosbahn 31 zwischen jeweils 2 benachbarten Verbindungslinien 14 quer zur Bahn 31 geschnitten, um ein einzelnes Kleidungsstück 1 abzutrennen. Schließlich wird in Schritt (V) das einzelne Kleidungsstück 1 erhalten. Während die jeweiligen Schritte (I) bis (V) hier als kontinuierlicher Vorgang dargestellt sind, können diese Schritte auch in geeignetem Abstand zueinander erfolgen. Auch ist es möglich, getrennte Endlosbahnen vorzusehen, die als Vorderabschnitt 10 bzw. Hinterabschnitt 11 dienen sollen, und diese Bahnen getrennt voneinander zuzuliefern, aufeinander zu legen und in dem der Linie 13A entsprechenden Bereich miteinander zu verbinden.

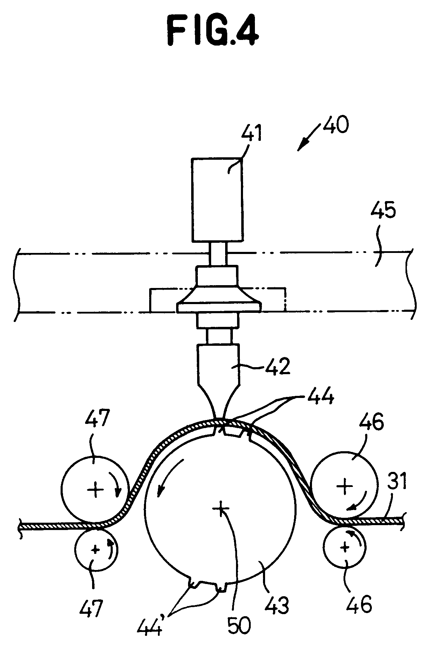

Eine in Fig. 4 bis 6 dargestellte Ultraschall-Schweißeinrichtung 40, die zum Anbringen der beiden Verbindungslinien 14 dient, wird nachfolgend beschrieben. Fig. 4 ist eine Seitenansicht der Schweißeinrichtung. Die Schweißeinrichtung 40 umfaßt einen Ultraschallwellengenerator 41, einen direkt mit dem Generator 41 verbundenen Schallkopf 42, eine drehbare Walze 43, die dem Schallkopf 42 gegenüberliegend angeordnet ist, und jeweils paarweise an der Umfangsfläche der Walze 43 angeordnete Stempel 44, 44'. Der Generator 41 ist an einem höhenverstellbaren Preßarm 45 gehaltert. Weiter umfaßt die Schweißeinrichtung 40 zwei Führungswalzenpaare 46, 47, die die Endlosbahn 31 zwischen den Schallkopf 42 und die Stempel 44 oder 44' führen. Die Bedingungen zur Erzeugung der Ultraschallwellen wie auch die Preßbedingungen des Armes 45 können in geeigneter Weise gewählt werden, um die Endlosbahn 31 mit Schweißstellen zu versehen, die keine hautreizende Wirkung haben.

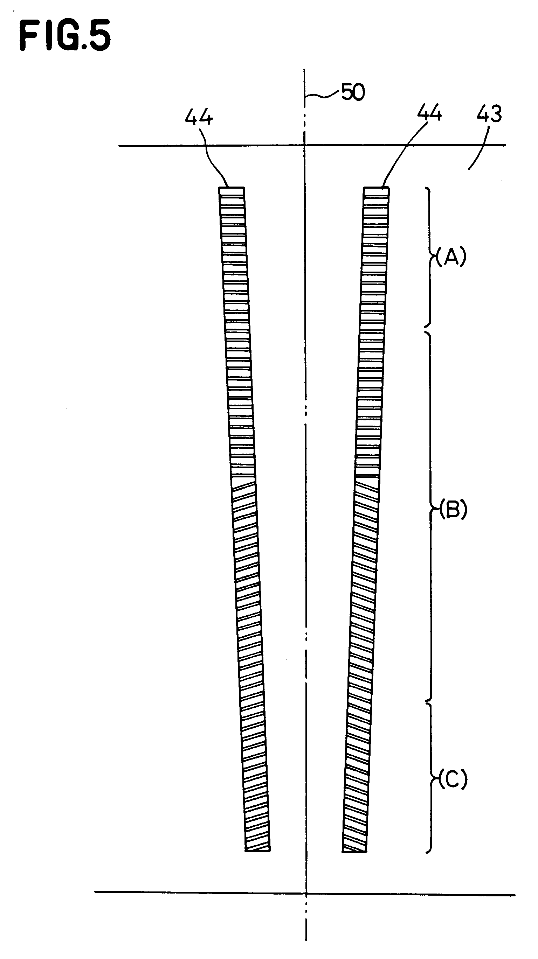

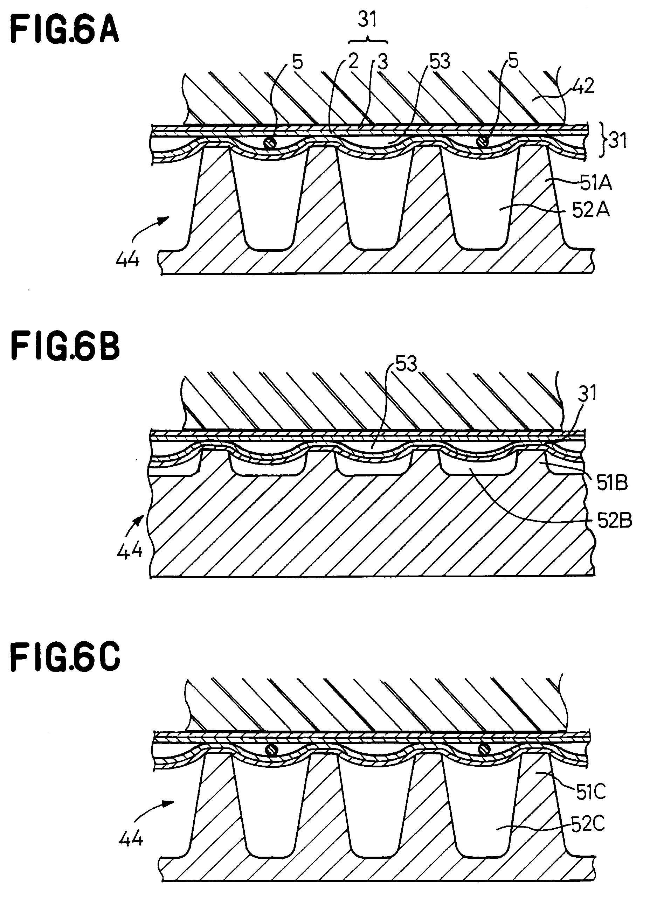

Fig. 5, 6A, 6B und 6C sind eine Draufsicht bzw. Seitenansichten der Stempel 44. Fig. 5 zeigt die paarweise angeordneten Stempel 44 auf der Umfangsfläche der drehbaren Walze 43, die symmetrisch, jedoch in kleinem Winkel bezüglich einer Achse 50 der Walze 43 angeordnet sind. Das andere Stempelpaar 44' entspricht dem Stempelpaar 44. In dieser Weise kann die Anzahl der Stempelpaare in geeigneter Weise in Abhängigkeit von der Größe der Walze 43 gewählt werden. Der Schallkopf 43 hingegen ist parallel zur Achse 50 angeordnet und kreuzt daher die Stempel 44 in der Weise, daß ein Bereich, in dem der Schallkopf 43 und die Stempel 44 quer gegeneinander gepreßt werden, wobei die Endlosbahn 31 dazwischen liegt, im Vergleich zu dem Fall, in dem der Schallkopf und die Stempel parallel zueinander angeordnet sind, beträchtlich reduziert ist. Dadurch ist die auf dem Schallkopf 42 lastende, für die Durchführung eines ordnungsgemäßen Schweißvorganges erforderliche Last relativ reduziert und der verwendete Ultraschallwellengenerator 41 kann auch in kleinerer Ausführung gewählt werden. Damit sind die Herstellungskosten der Einrichtung 40 in vorteilhafter Weise verringert. Die Stempel 44 eines jeden Paares können nahe aneinander liegend angeordnet sein, so daß ein nutzloser Bereich der Endlosbahn 31, der zwischen benachbarten Kleidungsstücken 1 notwendigerweise vorhanden ist, möglichst gering gehalten wird.

Fig. 6A, 6B und 6C sind vergrößerte Seitenansichten der Stempel 44. Fig. 6A, 6B und 6C entsprechen den Abschnitten (A), (B) bzw. (C) in Fig. 5. Jeder Stempel 44 in den Figuren umfaßt mehrere Vorsprünge 51A, 51B, 51C, die durch Einschnitte 52A, 52B, 52C von bestimmter Tiefe und Anordnung voneinander getrennt sind. Die Anordnung der Vorsprünge wie auch der Vertiefungen ist vom gewünschten Verbindungsmuster abhängig. Während es allgemein wichtig ist, daß die Vertiefungen relativ flach sind, um ein übermäßiges Durchhängen bzw. eine übermäßige Lose der Endlosbahn 31 zu vermeiden, sind die Vertiefungen 52A, 52C tiefer dargestellt als die Vertiefung 52B, so daß die elastischen Elemente, beispielsweise das elastische Element 5, vollständig in diesen aufgenommen werden können.

Wie ebenfalls in Fig. 6A, 6B und 6C dargestellt, pressen der Schallkopf 42 und der Stempel 44 die Endlosbahn 31 zwischen sich, um so den gewünschten Schweißvorgang durchzuführen. In Bereichen, die über den Vertiefungen 52A, 52B, 52C liegen, wird kein Schweißvorgang durchgeführt, so daß daher die auf diese Weise übereinandergefaltete und verschweißte Endlosbahn 31 mit Spalten 53 versehen ist. Die Spalten 53 wirken als Belüftungsöffnungen, die zwischen dem Vorderabschnitt 10 und dem Hinterabschnitt 11 angeordnet sind, und durch die das innere des Kleidungsstückes 1 mit der Außenumgebung in Verbindung steht. Die elastischen Elemente 5, 6 können vollständig in den Vertiefungen aufgenommen werden und unterliegen nicht der Preßwirkung, so daß daher die Endlosbahn 31 zu einer gleichmäßigen Stärke zusammengepreßt und unter stabilen Bedingungen verschweißt werden kann. Die Stempel 44 können von einer zugehörigen Heizung in geeigneter Weise erwärmt werden, um die für den Schweißvorgang erforderliche Zeit zu verkürzen. Eine derartige Heizung ist nicht dargestellt.

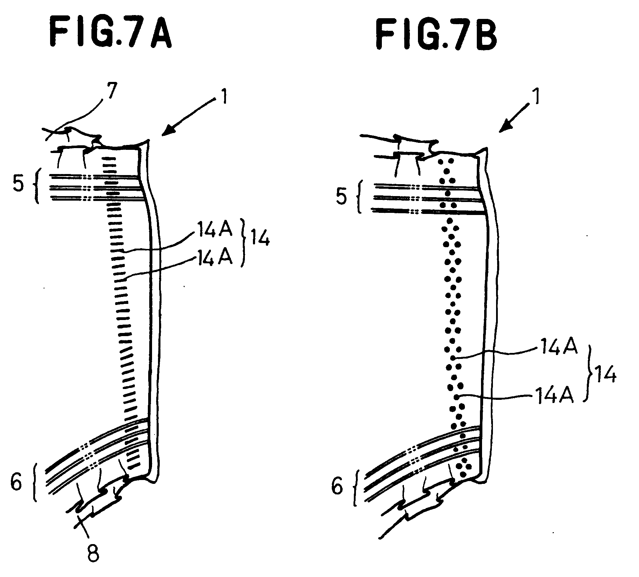

Fig. 7A und 7B sind perspektivische Teilansichten des Kleidungsstückes 1 zur Darstellung von Einzelheiten der Verbindungslinien 14. Die in Fig. 7A gezeigte Verbindungslinie 14 umfaßt mehrere im Abstand zueinander und parallel zueinander angeordnete, im wesentlichen rechteckige Schweißbereiche 14A. Die Ausrichtung dieser Schweißbereiche 14A ist jedoch in dem der Hüftöffnung 7 benachbarten Bereich unterschiedlich von der Ausrichtung in dem den Beinöffnungen benachbarten Bereich. Im einzelnen sind dabei einige Schweißbereiche 14A parallel zum elastischen Element 5 im Bereich an der Hüftöffnung 7 ausgerichtet und in dem der jeweiligen Beinöffnung 8 benachbarten Bereich sind einige Schweißbereiche 14A parallel zu dem im wesentlichen halbkreisförmigen elastischen Element 6 ausgerichtet. Die jeweiligen elastischen Elemente 5, 6 liegen klarerweise in nicht verschweißten Bereichen, die von jeweils benachbarten Schweißbereichen 14A begrenzt werden. Die in Fig. 7B gezeigte Verbindungslinie umfaßt mehrere verteilte, im wesentlichen kreisförmige Schweißbereiche 14A, und die jeweiligen elastischen Elemente 5, 6 sind in Abständen angeordnet, die zwischen diesen Schweißbereichen 14A liegen.

Sowohl die obere Lage 2 als auch die untere Lage 3 des Kleidungsstückes 1 enthalten thermisch verschweißbares Material, das beispielsweise aus thermoplastischen Synthetikfasern bzw. aus Geweben oder Vliesstoff daraus bestehen kann, aus thermoplastischer Folie oder Elastomerfolie. Von diesen ist Vliesstoff mit warmgekräuselten Verbundfasern hinsichtlich des Griffs und der Dehnbarkeit besonders bevorzugt. Diese Gewebe, Vliesstoffe und Folien können einzeln oder in Verbindung miteinander verwendet werden. So kann beispielsweise dehnbare Folie auf dehnbaren Vliesstoff aufgebracht werden, wobei der Vliesstoff die Außenseite bildet und diese Anordnung als untere Lage dient. Die elastischen Elemente 5, 6 können aus Naturkautschuk oder synthetischem Gummi bestehen. Der Absorbtionskern 4 kann aus zerkleinerter Pulpe bestehen, die mit einem hoch wasserabsorbierenden Polymerpulver gemischt ist und anschließend in die gewünschte Form gepreßt ist.

Der Vorder- und Hinterabschnitt, der jeweils die thermisch schweißbares Material enthaltende obere und untere Lage umfaßt, werden übereinander gelegt und anschließend kontinuierlich der Ultraschall-Schweißeinrichtung zugeführt, die Stempel aufweist, die jeweils mehrere im Abstand zueinander angeordnete Vorsprünge aufweist, wodurch die Kleidungsstücke kontinuierlich entlang den seitlich gegenüberliegenden Hüftseiten mit Verbindungslinien versehen werden, die jeweils ein unterbrochenes Verbindungsmuster aufweisen.

Die oberen und unteren Lagen werden miteinander verschweißt, wobei die elastischen Elemente, die um die Hüftöffnung und/oder die Beinöffnungen angebracht sind, in den Vertiefungen der jeweiligen Stempel angeordnet sind, so daß die elastischen Elemente den Schweißvorgang nicht behindern. Auf diese Weise wird das Schweißen unter stabilen Bedingungen durchgeführt. Damit sind die Verbindungslinien in ansprechender Erscheinungsform und mit nicht hautreizendem Griff leichter zu erzielen.

Die Anordnung, daß der Schallkopf parallel zur Walzenachse angeordnet ist, während die Stempel in einem bestimmten Winkel bezüglich dieser Achse vorgesehen sind, verringert in vorteilhafter Weise den Bereich, in dem der Schallkopf und die Stempel gegeneinander gepreßt werden, wodurch entsprechend die auf den Schallkopf treffende Last verringert wird.

Die Stempel können erwärmt werden, um die für den gewünschten Schweißvorgang erforderliche Zeit zu verringern.

## Patentansprüche

1. Verbindungsverfahren in einem kontinuierlichen Herstellungprozeß für höschenartige Kleidungsstücke (1), umfassend die Schritte des Übereinanderlegens von Vorder- und Hinterabschnitten des Kleidungsstückes mit jeweils wenigstens einer wasserdurchlässigen oberen Lage (2) und einer wasserundurchlässigen unteren Lage (3), die thermisch verschweißbares Material enthalten, und anschließend des Bildens von luftdurchlässigen Verbindungslinien (14) entlang seitlich gegenüberliegenden Hüftseiten des Kleidungsstückes, wobei
zur Durchführung des Verbindens eine Ultraschall-Schweißeinrichtung (40) mit einen Schallkopf (41) und einem Stempel (44, 44') verwendet wird, deren Länge jeweils im wesentlichen der Länge der Verbindungslinien (14) entspricht, die entlang den seitlich gegenüberliegenden Hüftseiten ausgebildet werden,
der Schallkopf (41) quer zur Zulieferrichtung der oberen und unteren Lage (2, 3) vorgesehen ist,
die Stempel (44, 44') gegenüber dem Schallkopf (41) an der Umfangsfläche einer Walze (43) vorgesehen sind, die in der Zulieferrichtung der oberen und unteren Lage (2, 3) drehbar ist,
die Stempel (44, 44') mehrere Vorsprünge (51A, 51B, 51C) umfassen, die im Abstand zueinander angeordnet sind, so daß sie einem vorgegebenen Verbindungsmuster entsprechen, und
die übereinander gelegten Vorder- und Hinterabschnitte des Kleidungsstückes (1) der Ultraschall-Schweißeinrichtung (40) zwischen dem Schallkopf (41) und den Stempeln (44, 44') kontinuierlich zugeliefert werden, so daß die Verbindungslinien (14) mit dem Verbindungsmuster kontinuierlich entlang den seitlich gegenüberliegenden Hüftseiten ausgebildet werden.

2. Verfahren nach Anspruch 1,
wobei das Kleidungsstück (1) elastische Elemente (5, 6) umfaßt die um eine Hüftöffnung und/oder Beinöffnungen angeordnet sind, und die elastischen Elemente während des Ultraschall-Schweißvorganges zwischen den Vorsprüngen (51A, 51B, 51C) aufgenommen werden.

3. Ultraschall-Schweißeinrichtung für einen kontinuierlichen Herstellungsprozeß von höschenartigen Kleidungsstücken (1), der die Schritte des Übereinanderlegens von Vorderabschnitten und Hinterabschnitten des Kleidungsstückes mit jeweils wenigstens einer wasserdurchlässigen oberen Lage (2) und einer wasserundurchlässigen unteren Lage (3), und des anschließenden Bildens von luftdurchlässigen Verbindungslinien (14) entlang seitlich gegenüberliegenden Hüftseiten des Kleidungsstückes umfaßt mit einem Schallkopf (41) mit einer im wesentlichen der Länge jeder Verbindungslinie (14), die entlang den jeweiligen seitlich gegenüberliegenden Hüftseiten auszubilden ist, entsprechenden Länge, wobei der Schallkopf (41) quer zur Zulieferrichtung der oberen und unteren Lagen (2, 3) angeordnet ist, und mit Stempeln (44, 44') gegenüber den Schallkopf (41) an der Umfangsfläche einer in Zulieferrichtung der oberen und unteren Lage drehbaren Walze (43) mit mehreren Vorsprüngen (51A, 51B, 51C), die im Abstand zueinander angeordnet sind, so daß sie einem vorgegebenen Verbindungsmuster entsprechen.

4. Ultraschall-Schweißeinrichtung nach Anspruch 3, wobei der Schallkopf (41) parallel zur Achse (50) der Walze (43) angeordnet ist, während die Stempel (44, 44') paarweise auf der Umfangsfläche der Walze (43) in einem Winkel zur Walzenachse vorgesehen sind.

5. Ultraschall-Schweißeinrichtung nach Anspruch 3, wobei ein Abstand (52A, 52B, 52C) zwischen benachbarten Vorsprüngen (51A, 52A, 52C) in einem vorgegebenen Bereich jedes Stempels (44, 44') größer ist als die Breite eines elastischen Elementes (5, 6), das um die Hüftöffnung und/oder die Beinöffnungen angebracht ist.

6. Ultraschall-Schweißeinrichtung nach Anspruch 3, wobei in Verbindung mit den Stempeln (44, 44') eine Heizeinrichtung vorgesehen ist.

## Claims

1. Attaching method in a continuous manufacturing process for pants-type wearing articles (1) including the steps of laying front and rear bodies of said wearing article each comprising at least a water-permeable topsheet (2) and a water-impermeable backsheet (3) which contain thermally weldable material one on another and then forming air-permeable attaching lines (14) along laterally opposite waist sides of said wearing article, wherein
a supersonic welder (40) used to perform said attaching includes a horn (41) and an anvil (44, 44') both of which have lengths substantially corresponding to that of the attaching lines (14) to be formed along said laterally opposite waist sides;
said horn (41) is provided transversely of a feeding direction of said top- and backsheet;
said anvils (44, 44') are opposed to said horn (41) and provided on a peripheral surface of a drum (43) rotatable in the feeding direction of said top- and backsheet (2, 3);
said anvils (44, 44') comprise a plurality of projections (51A, 51B, 51C) arranged in a distance to each other so as to correspond to a given attaching pattern; and
said front and rear bodies of the wearing article (1) laid one on another are continuously fed to said supersonic welder (40) between said horn (41) and said anvils (44, 44') so that the attaching lines (14) with said attaching pattern are continuously formed along the laterally opposite waist sides.

2. Method as claimed in claim 1,
wherein said wearing article (1) includes elastic members (5, 6) attached around a waist-opening and/or leg-openings and said elastic members are received between said projections (51A, 51B, 51C) during the supersonic welding process.

3. Supersonic welder for a continuous manufacturing process for pants-type wearing articles (1) including the steps of laying front and rear bodies of said wearing article each comprising at least a water-permeable topsheet (2) and a water-impermeable backsheet (3) one on another and then forming air-permeable attaching lines (14) along laterally opposite waist sides of said wearing article, having a horn (41) which has a length substantially corresponding to that of each attaching line (14) to be formed along each of the laterally opposite waist sides, said horn (41) being provided transversely of the feeding direction of said top- and backsheet (2, 3), and anvils (44, 44') provided opposed to said horn (41) on a peripheral surface of a drum (43) rotatable in the feeding direction of said top- and backsheet comprising a plurality of projections (51A, 51B, 51C) arranged in a distance to each other so as to correspond to a given attaching pattern.

4. Supersonic welder as claimed in claim 3,
wherein said horn (41) is provided in parallel with an axis (50) of said drum (43) while said anvils (44, 44') are provided in pairs on the peripheral surface of said drum (43) at an angle with respect to said drum axis.

5. Supersonic welder as claimed in claim 3,
wherein a clearance (52A, 52B, 52C) between adjacent projections (51A, 51B, 51C) in a predetermined area of each anvil (44, 44') is larger than the width of an elastic member (5, 6) attached around said waist-opening and/or leg-openings.

6. Supersonic welder as claimed in claim 3,
wherein there is provided a heater in association with said anvils (44, 44').

## Revendications

1. Procédé de raccordement intervenant dans un processus de confection continu pour vêtements (1) du type petite culotte, comprenant les opérations consistant à superposer des parties avant et arrière du vêtement comprenant chacune au moins une couche supérieure (2) perméable aux liquides et une couche inférieure (3) imperméable aux liquides qui contiennent une matière thermosoudable, puis à former des lignes de jonction (14) laissant passer l'air, le long de côtés de hanches latéralement opposés du vêtement, procédé selon lequel
- pour exécuter le raccordement, on utilise un dispositif de soudage par ultrasons (40) équipé d'une tête sonique (41) et d'un poinçon (44, 44'), dont la longueur correspond respectivement, en substance, à la longueur des lignes de jonction (14) qui sont formées le long des côtés de hanches latéralement opposés,
- la tête sonique (41) est prévue transversalement à la direction d'amenée des couches supérieure et inférieure (2, 3),
- les poinçons (44, 44') sont prévus en regard de la tête sonique (41), sur la face périphérique d'un cylindre (43) qui tourne dans le sens d'amenée des couches supérieure et inférieure (2, 3),
- les poinçons (44, 44') comprennent plusieurs saillies (51A, 51B, 51C) disposées à distance les unes des autres, en sorte qu'elles correspondent à une trame de raccordement prédéterminée, et
- les parties avant et arrière superposées du vêtement (1) sont amenées en continu au dispositif de soudage par ultrasons (40), entre la tête sonique (41) et les poinçons (44, 44'), de sorte que les lignes de jonction (14) conformes à la trame de raccordement soient formées en continu le long des côtés de hanches en opposition latérale.

2. Procédé suivant la revendication 1, selon lequel le vêtement (1) comprend des éléments élastiques (5, 6) mis en place autour d'une ouverture de hanches et/ou d'ouvertures de passage des jambes, et les éléments élastiques sont engagés entre les saillies (51A, 51B, 51C) pendant l'opération de soudage par ultrasons.

3. Dispositif de soudage par ultrasons destiné à un processus de confection continu de vêtements (1) du type petite culotte comprenant les opérations consistant à superposer des parties avant et arrière du vêtement possédant chacune au moins une couche supérieure (2) perméable à l'eau et une couche inférieure (3) imperméable à l'eau, puis à former des lignes de jonction (14) laissant passer l'air le long de côtés de hanches latéralement opposés du vêtement, dispositif comprenant une tête sonique (41) d'une longueur correspondant, sensiblement, à la longueur de chaque ligne de jonction (14) qui doit être formée le long de chacun des côtés de hanches latéralement opposés, ladite tête sonique (41) étant disposée transversalement à la direction d'amenée des couches supérieure et inférieure (2, 3), et avec des poinçons (44, 44') disposés en regard de la tête sonique (41) sur la face périphérique d'un cylindre (43) tournant dans le sens d'amenée des couches supérieure et inférieure et pourvu d'une pluralité de saillies (51A, 51B, 51C) disposées à distance les unes des autres, pour correspondre à une trame de raccordement prédéterminée.

4. Dispositif de soudage par ultrasons suivant la revendication 3, dans lequel la tête sonique (41) est disposée parallèlement à l'axe (50) du cylindre (43), tandis que les poinçons (44, 44') sont prévus par paires sur la face périphérique du cylindre (43), en formant un angle avec l'axe du cylindre.

5. Dispositif de soudage par ultrasons suivant la revendication 3, dans lequel la distance (52A, 52B, 52C) séparant des saillies voisines (51A, 51B, 51C) est plus grande, dans une zone de chaque poinçon (44, 44') définie au préalable, que la largeur d'un élément élastique (5, 6) qui est mis en place autour de l'ouverture de hanches et/ou des ouvertures de passage des jambes.

6. Dispositif de soudage par ultrasons suivant la revendication 3, dans lequel un dispositif de chauffage est prévu en relation avec les poinçons (44, 44').
